# EUROPEAN PATENT APPLICATION

(11) **EP 4 147 728 A1**
(43) Date of publication of application: **15.03.2023**
(21) Application number: 20935355.6
(22) Date of filing: 09.05.2020
(51) Int. Cl.: A61L 2/14, A61L 2/26, A61L 2/24, G01D 21/02

(54) **ADJUSTABLE CONTINUOUS FLOW PLASMA DISINFECTION AND STERILIZATION METHOD, AND DISINFECTION AND STERILIZATION DEVICE CORRESPONDING TO SAME**

(71) Applicant: Disen Sterilization Technology (Xiaogan) Co., Ltd., Hubei 432000 (CN)
(72) Inventor: ZHANG, Linde, Hubei 432000 (CN); YAN, Xiangjun, Hubei 432000 (CN)
(74) Representative: Ipey
(86) International application number: PCT/CN2020/089416
(87) International publication number: WO 2021/226751

(57) **Abstract**

Provided are an adjustable continuous-flow plasma disinfection and sterilization method and a disinfection and sterilization device. The adjustable continuous-flow plasma disinfection and sterilization method includes: (1) exciting atmospheric-pressure plasmas in a disinfection and sterilization chamber featuring an adjustable inlet-outlet pressure difference; (2) adjusting the inlet-outlet pressure difference of the disinfection and sterilization chamber after the atmospheric-pressure plasmas become stable, so as to adjust the plasma density in the chamber; (3) continuously feeding microorganism carriers to be disinfected and sterilized into the disinfection and sterilization chamber for disinfection or sterilization; and (4) continuously discharging the carriers out of the disinfection and sterilization chamber to complete disinfection or sterilization. The method allows continuous work under atmospheric pressure, and can disinfect or sterilize closed, semi-open or open spaces.

## Description

### TECHNICAL FIELD

The present invention relates to the field of disinfection and sterilization. In addition, the present invention also relates to devices and instruments in the field of disinfection and sterilization.

### BACKGROUND

Bacteria, fungi, viruses and other microorganisms are important components of the natural environment, and the transformation, degradation and synthesis of a large number of substances in the natural environment depend on these microorganisms. Genetically modified microorganisms, such as E.coli transplanted with penicillin synthesis genes, can directionally transform a carbon source, nitrogen source and sulfur source in a fermentation broth into penicillin, a micromolecule with a specific structure. However, the influence of microorganisms on human beings can be harmful, even fatal and intricate in some cases. Bio-safety problems caused by superbacteria such as methicillin-resistant Staphylococcus aureus (MRSA) and multidrug-resistant Streptococcus pneumoniae (MDRSP), common bacteria of Acinetobacter, Pseudomonadaceae and Enterobacteriaceae families, extremely harmful viruses such as SARS-CoV-2 and Ebola, and fungi with strong drug resistance and high lethality such as Candida auris have become the greatest threat to human health. In the in vivo environment, it is hard to inhibit the growth of these bacteria, viruses and fungi with conventional antibiotics or antiviral drugs; and in the in vitro environment, these microorganisms are highly contagious, so traditional disinfection methods are barely effective to inactivate these microorganisms. A large number of public security incidents have been caused thereby, which have greatly affected the economic operation and social organization of human society. In addition, the fact that microorganisms are subject to mutation and antagonism inheritance makes existing antibiotics and disinfection techniques less effective or even lose their efficacy completely. Thus, the fight between human beings and these pathogenic microorganisms is bound to be a long-term battle, and disinfection techniques with broader spectra and stronger efficacy are needed to deal with the invasion of these microorganisms.

For this reason, to improve disinfection techniques which are used to kill or eliminate pathogenic microorganisms on media by chemical or physical methods to realize zero transmission and infection, technological breakthroughs in basic research fields and transformation in microbial research fields are generally required. Traditional disinfection techniques include mechanical disinfection, thermal disinfection, radiation disinfection and chemical disinfection. Mechanical disinfection is to realize environmental disinfection through running water washing, aseptic airflow and laminar flow scouring and the like. Thermal disinfection refers to the heat treatment of objects to be disinfected by different heating means such as burning, boiling, flowing steam or high-pressure steam, and dry heat, so as to kill bacteria. The heating temperature is generally 100-200°C, and the duration varies from several minutes to several hours, depending on the objects to be disinfected. Radiation disinfection is mainly achieved by ionizing radiation and non-ionizing radiation. Non-ionizing radiation includes ultraviolet, infrared and microwave radiation, and ionizing radiation includes cathode ray and Co-60 source radiation. Chemical disinfection realizes environmental disinfection mainly through different disinfectants, including phenol, ethanol, ethylene oxide, bleaching powder, chlorine dioxide, sodium dichloroisocyanurate, peracetic acid, hydrogen peroxide, glutaraldehyde, and quaternary ammonium salt, generally involving spraying, atomizing and scrubbing.

Generally speaking, the above-mentioned disinfection techniques are suitable for different scenes and environments, and their disinfection levels vary with different microorganism killing rates in the environment. A disinfection level where all microorganisms are killed or removed can be called sterilization.

Ethylene oxide disinfection, high-pressure steam disinfection, dry heat disinfection and ionizing radiation disinfection, among other techniques mentioned above, can all reach the sterilization level. Sterilization is an absolute concept, which means killing all microorganisms. Sterilized articles can directly enter the sterile tissue of the human body without infection. Therefore, sterilization can be regarded as the most thorough disinfection. However, it is hard to reach such a level in reality. Therefore, the international standard stipulates that the survival probability of microorganisms causing contamination to articles must be reduced to 10-6 (sterility assurance level) in the sterilization process. In other words, only when the target microorganism killing rate reaches 99.9999% can the technical requirements of sterilization be met. A disinfection technique reaching the sterilization level can also be regarded as the most effective disinfection technique.

However, techniques that can realize sterilization are often limited in application. The most basic sterilization is high-pressure steam sterilization, which involves a treatment of 30 min or longer in steam at a temperature as high as 120°C. From all these disinfection techniques, it can be seen that a disinfection technique often has to make compromises in other aspects to reach the sterilization level, for example, it cannot be widely implemented, or it may cause great harm to the human body. On the contrary, a disinfection technique too mild cannot reach the sterilization level. Therefore, it is easy to speculate that an ideal disinfection technique should have the following characteristics.

The disinfection intensity should be as high as possible, and the microorganism killing rate can reach 99.9999%, that is, the sterilization level; the disinfection speed should be as fast as possible, time consumed should be as short as possible, post-treatment is not required, and if there are any residues, they should be harmless; disinfection of a large-area environment can be realized, especially an aerosol environment, without damaging instruments and articles in the environment; and the cost of consumables in the disinfection process should be as low as possible, and no or little manpower should be introduced.

The above characteristics are just what the existing disinfection techniques fail to have at the same time. However, if a new technology transformed from basic research is to be introduced into the field of disinfection and sterilization, it should be as close to these indicators and characteristics as possible. At present, the most ideal disinfection technique is plasma disinfection technique.

The mainstream plasma disinfection technique still uses vacuum or negative-pressure plasmas for disinfection. This means that such plasmas are vacuum negative-pressure plasmas, which are generally low-temperature plasmas. The atom temperature of low-temperature plasmas is low in general, between 10² K and 10³ K, the electron temperature is generally 10⁴ K, and the ionization degree is low, generally smaller than 1%. Under such conditions, only a small number of particles in cold plasmas are ionized, and the excited state of the formed plasmas is low. Therefore, other additives are often needed to make the active components in the plasmas have high activity. For example, by injecting hydrogen peroxide and allowing the plasmas to assist in the ionization of hydrogen peroxide, high-concentration oxygen free radicals are obtained and a closed chamber is disinfected. The chamber is sealed under negative pressure for a period of time for disinfection. When disinfection is completed, atmospheric-pressure clean gas is introduced to restore the chamber to atmospheric pressure. For such disinfection technique where cold plasmas assist in the ionization of hydrogen peroxide, the pressure in the chamber generally ranges from several Pa to dozens of Pa, and the working temperature of about 35-45°C. This technique can realize nondestructive sterilization of ordinary instruments and polymer materials with a sterilization cycle of 30-60 minutes and efficiency much higher than other methods.

There are many mature applications of the low-temperature plasma disinfection technique by far. For example, Starrad 100S, a hydrogen peroxide plasma sterilizer approved by the FDA in 1997 by Johnson & Johnson, has entered the Chinese market since 2004 and has been widely accepted. However, this does not mean that the low-temperature plasma disinfection technique has reached the peak of disinfection techniques. The plasma disinfection technique still has some defects. The most direct problem is that such plasmas are negative-pressure or vacuum plasmas, which are not suitable for wide-range disinfection and sterilization. Even in a chamber, because there is no pressure difference in the plasma environment, the flow is blocked and it is hard to form a large-scale uniform plasma atmosphere. Once the size of the chamber increases, the corresponding vacuum requirement becomes higher and even harder to meet.

It can be said that the biggest obstacle to large-scale application of the plasma disinfection technique is the vacuum requirement. Therefore, a lot of research has been done to realize the formation of plasmas under normal pressure, that is, the formation of plasma torches. It is not easy to form stable plasmas under normal pressure, because the mean free path of particles under normal pressure is often small, and energy will be quickly transferred out through collisions between particles. Unless the degree of the initial non-equilibrium state is high, it is difficult to form stable plasmas. Atmospheric-pressure plasmas, especially surface-coupled plasmas, have higher energy feeding efficiency, a wider adjustable range of electron temperature and ion temperature, and higher energy density. Such atmospheric-pressure plasmas make it possible to apply the high-temperature atmospheric-pressure plasma technique to the field of disinfection.

### SUMMARY

### Technical problem

In view of this, the present invention proposes an adjustable continuous-flow plasma disinfection and sterilization method with superior performance and a corresponding disinfection and sterilization device.

### Solution to problem

### Technical solution

In one aspect, the present invention provides an adjustable continuous-flow plasma disinfection and sterilization method which includes the following steps.

Atmospheric-pressure plasmas are excited in a disinfection and sterilization chamber featuring an adjustable inlet-outlet pressure difference. After the atmospheric-pressure plasmas become stable, the inlet-outlet pressure difference of the disinfection and sterilization chamber is adjusted according to corresponding disinfection or sterilization requirements, so as to adjust the plasma density in the chamber. Then microorganism carriers to be disinfected and sterilized are continuously fed into the disinfection and sterilization chamber for disinfection and sterilization. After treatment, the carriers are discharged out of the disinfection and sterilization chamber, marking the completion of disinfection or sterilization of the carriers according to different microorganism killing rate requirements.

Further, the atmospheric-pressure plasmas may be capacitive coupling plasmas, inductive coupling plasmas, high voltage DC arcs, high voltage AC arcs, microwave plasmas and surface coupling induced plasmas.

Further, the plasma density of the atmospheric-pressure plasmas is 1.0×10³-1.0×10³ plasmas per cubic centimeter.

Further, the atom temperature of the atmospheric-pressure plasmas is 1.0×10⁰-1.0×10¹²K.

Further, the electron temperature of the atmospheric-pressure plasmas is 1.0×10⁰-1.0×10¹² K.

Further, microorganisms carried by the microorganism carrier include bacteria (and spores thereof), archaea, fungi, actinomycetes, protozoa, algae, viruses, mycoplasma, chlamydia, viroids, virusoids and prions.

Further, the microorganism killing rate ranges from 90% to 99.9999%.

Further, exciting atmospheric-pressure plasmas in a disinfection and sterilization chamber featuring an adjustable inlet-outlet pressure difference specifically includes the following steps. At 1S1, a working medium gas is introduced into an atmospheric-pressure plasma unit, and after the atmospheric-pressure plasmas are saturated with the working medium gas, energy is supplied to a plasma power source until the plasmas are successfully ignited to form a plasma torch. At 1S2, on the basis of ensuring that the plasmas are kept stable, the input power of the plasma source is gradually adjusted until the input power of the plasmas reaches target power.

Further, the working medium gas in 1S1 includes one or a mixture of hydrogen, oxygen, nitrogen, artificial air, helium, neon, argon, krypton, xenon, chlorine, fluorine, bromine vapor, hydrogen fluoride, hydrogen chloride, hydrogen iodide, hydrogen bromide, nitrogen dioxide, nitrous oxide, nitrogen trifluoride, carbon monoxide, carbon dioxide, ammonia, sulfur hexafluoride, carbon tetrafluoride, silane gas, germane or organic gas.

Further, the power of the plasma power source in 1S1 is 0.5 W-100 kW.

Further, the way of gradually adjusting the input power in 1S2 includes: first increasing the power and then decreasing the power to the target power, or gradually adding the power to the target power.

Further, adjusting the inlet-outlet pressure difference of the disinfection and sterilization chamber according to corresponding disinfection or sterilization requirements after the atmospheric-pressure plasmas become stable, so as to adjust the plasma density in the chamber specifically includes the following steps. At 2S1, on the basis of ensuring that the plasmas are kept stable, an outlet pressure of the disinfection and sterilization chamber is adjusted by means of an outlet pressure adjustment module in a pressure adjustment unit connected to the disinfection and sterilization chamber. At 2S2, on the basis of ensuring that the plasmas are kept stable, an inlet pressure of the disinfection and sterilization chamber is adjusted by means of an inlet pressure adjustment module in the pressure adjustment unit of the disinfection and sterilization chamber.

Further, the inlet-outlet pressure difference of the disinfection and sterilization chamber is 0 Pa-120 MPa.

Further, the inlet pressure of the disinfection and sterilization chamber is 0.0000001 Pa- 120 Mpa.

Further, the outlet pressure of the disinfection and sterilization chamber is 0.0000001 Pa-120 Mpa.

Further, the outlet pressure adjustment module in 2S1 includes one or more of a vortex fan, a centrifugal fan, an axial fan, a negative pressure Roots blower, a venturi tube, an air compressor, a gas decompression pump, a piston compressor, a jet vacuum pump, a screw vacuum pump, a liquid ring vacuum pump, a rotary vane vacuum pump, a claw vacuum pump, a Roots vacuum pump, a reciprocating vacuum pump, a molecular pump, a diffusion pump, an ion transport pump, an adsorption pump, a sublimation pump or a cryogenic pump.

Further, the inlet pressure adjustment module in 2S2 includes one or more of a vortex fan, a centrifugal fan, an axial fan, a Roots blower, a venturi tube, a gas booster pump, a piston compressor or a gas cylinder.

Further, continuously feeding microorganism carriers to be disinfected and sterilized into the disinfection and sterilization chamber for disinfection or sterilization specifically includes the following steps. At 3S1, the microorganism carriers are fed into the disinfection and sterilization chamber at a certain speed, and the movement speed and form of the microorganism carriers in the disinfection and sterilization chamber are adjusted according to a size of the disinfection and sterilization chamber and the power of a plasma source, so as to ensure that the microorganism carriers can stay in the disinfection and sterilization chamber for an amount of time that meets disinfection or sterilization requirements.

Further, the microorganism carrier in 3S1 may be an airflow carrier, a liquid flow carrier, a multiphase flow carrier or a solid carrier with a conveying movement mechanism.

Further, when the microorganism carrier is an airflow carrier, the movement speed thereof in the disinfection and sterilization chamber is measured as a flow rate of 0.0001-100000 m³/min.

Further, when the microorganism carrier is a liquid flow carrier, the movement speed thereof in the disinfection and sterilization chamber is measured as a flow rate of 0.0001-100000 m³/min.

Further, when the microorganism carrier is a multiphase flow carrier, the movement speed thereof in the disinfection and sterilization chamber is measured as a flow rate of 0.0001-100000 m³/min.

Further, when the microorganism carrier is a solid carrier with a conveying movement mechanism, the movement speed thereof in the disinfection and sterilization chamber is measured as a linear speed of 0.0001-60 m/min.

Further, the movement form of the microorganism carriers in the disinfection and sterilization chamber in 3 S 1 includes one or more of linear movement, spiral movement, vortex movement, rotary movement, reciprocating movement or random movement.

Further, the duration of stay that meets disinfection or sterilization requirements in the disinfection and sterilization chamber in 3S1 is 0.5 s-24 h.

Further, discharging the carriers out of the disinfection and sterilization chamber after treatment, which marks the completion of disinfection or sterilization of the carriers according to different microorganism killing rate requirements, specifically includes the following steps. At 4S1, after the microorganism carriers has stayed in the disinfection and sterilization chamber for an amount of time that meets disinfection or sterilization requirements, the microorganism carriers are discharged out of the disinfection and sterilization chamber to complete the disinfection process.

In another aspect, the present invention also provides a disinfection and sterilization device which includes a disinfection and sterilization chamber unit, a pressure adjustment unit, an atmospheric-pressure plasma unit, a microorganism carrier conveying unit, a state measurement and control unit and a temperature control unit. The disinfection and sterilization chamber unit contains an ignition module of the atmospheric-pressure plasma unit, and an inlet and outlet of the disinfection and sterilization chamber unit communicate with the pressure adjustment unit, thereby realizing pressure adjustment of the disinfection and sterilization chamber. The microorganism carrier conveying unit communicates with the pressure adjustment unit, such that microorganism carriers can be fed into the disinfection and sterilization chamber under different pressure environments and discharged after disinfection. The temperature control unit is connected to the disinfection and sterilization chamber unit, the atmospheric-pressure plasma unit and the microorganism carrier conveying unit, so as to adjust the working temperature of the above units and realize constant temperature. The state measurement and control unit controls the states of the disinfection and sterilization chamber unit, the atmospheric-pressure plasma torch unit, the microorganism carrier conveying unit and the pressure adjustment unit, so as to continuously measure, control and adjust the disinfection process.

Further, the disinfection and sterilization chamber unit includes a disinfection and sterilization chamber module, an optical measurement interface, an electrical measurement interface and a temperature measurement interface.

Further, the structure of the disinfection and sterilization chamber module may be one or more of a rectangular chamber structure, a cylindrical chamber structure, a spherical chamber structure, a conical chamber structure, an ellipsoidal chamber structure, a parabolic chamber structure, a hyperboloid chamber structure, a spiral chamber structure, a volute chamber structure or a special-shaped chamber structure.

Further, the pressure adjustment unit includes an outlet pressure adjustment module, an inlet pressure adjustment module, pressure measurement interfaces and flow measurement interfaces. Here, the outlet pressure adjustment module and the inlet pressure adjustment module are each provided with a pressure measurement interface and a flow measurement interface.

Further, the atmospheric-pressure plasma unit includes a plasma source power module, a working medium inlet module, an ignition module and a power feedback measurement interface. Here, the plasma source ignition module is arranged in the disinfection and sterilization chamber module, the working medium inlet module is installed at a rear end of the ignition module, and the plasma source power module is provided with the power feedback measurement interface and can be monitored by the state measurement and control unit.

Further, the temperature control unit includes a heat dissipation module and a heat exchange module. Here, the heat exchange module is connected to the disinfection and sterilization chamber unit, the atmospheric-pressure plasma unit and the microorganism carrier conveying unit to ensure that the heat generated during the operation of the above units can be quickly transferred out. Meanwhile, the heat exchange module is connected to the heat dissipation module to ensure that the heat can quickly contact with the environment or a refrigerant/heat medium, thus controlling the temperature.

Further, the microorganism carrier conveying unit includes an airflow conveying module, or a liquid flow conveying module, or a multiphase flow conveying module or a solid carrier module with a conveying movement mechanism.

Further, the state measurement and control unit includes a control panel, a master control module, an optical measurement module, an electrical measurement module, a temperature measurement module, a pressure measurement module, a flow measurement module and a plasma source power feedback measurement module

### Beneficial effects of the Invention

### Beneficial effects

Compared with the prior art, the disinfection and sterilization method can work under atmospheric pressure, and can realize sterilization and disinfection in open, semi-closed and closed areas by continuously treating fluids such as aerosol and multiphase flow in the environment, thus directly solving the space limitation problem of plasma disinfection. In addition, for continuous surface sterilization and disinfection, solid carrier modules under atmospheric pressure can be used to realize continuous feeding and discharging, so that surface disinfection of surgical instruments, implants and other materials can be carried out in large quantities.

The present invention further provides a corresponding disinfection and sterilization device, which also has the technical effects of the aforementioned disinfection and sterilization method.

### BRIEF DESCRIPTION OF DRAWINGS

### Description of drawings

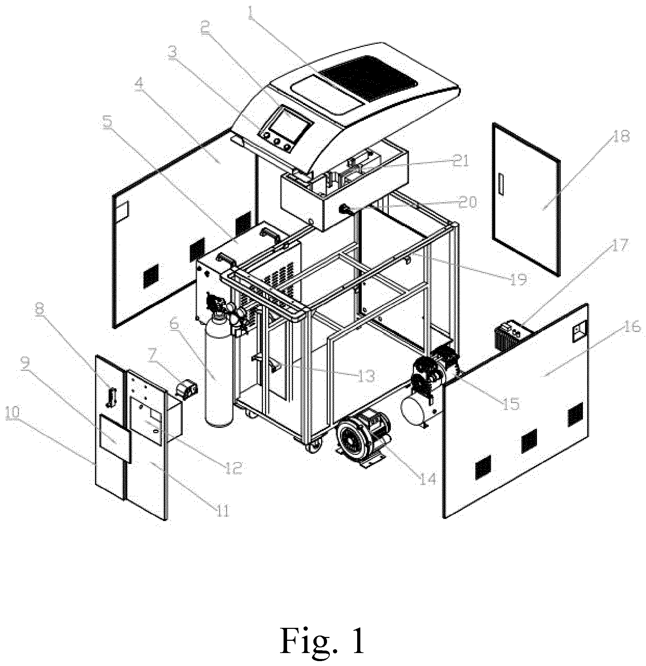

Fig. 1 is a schematic diagram of a specific implementation of a disinfection and sterilization device provided by the present invention.
   Here, the corresponding relationship between reference numerals and component names in Fig. 1 is as follows:
   Housing: 1: Top cover; 4&10&11&16: Framework plate; 19: Frame; 9: Access opening; 13: Fixing component;
   Disinfection and sterilization chamber unit 21: Disinfection and sterilization chamber module (rectangular chamber structure, including surface coupling induced plasma source ignition module, working medium inlet module, optical measurement interface and electrical measurement interface); 20: Temperature measurement interface;
   Pressure adjustment unit: 14: Fan as outlet pressure adjustment module (with pressure measurement interface and flow measurement interface); 15: Gas cylinder as inlet pressure adjustment module (with pressure measurement interface); 8: Flow measurement interface;
   Atmospheric-pressure plasma unit: 17: Plasma source power module and power feedback measurement interface; 6: Working medium inlet module
   Temperature control unit: 5: Temperature control unit;
   Microorganism carrier conveying unit: 7: Fluid conveying module;
   State measurement and control unit: 2&3: Control panel; 18: Master control module.

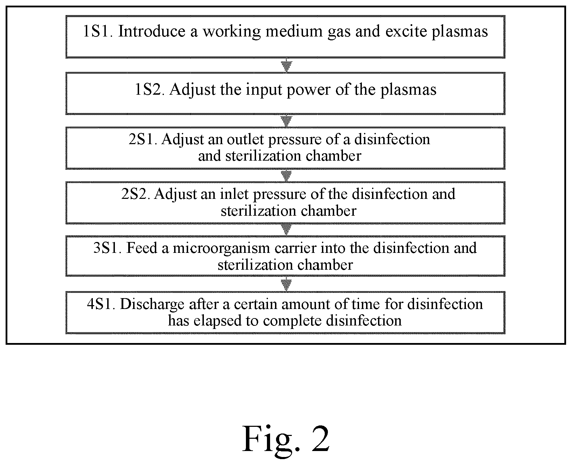

Fig. 2 is a simple flowchart.

### DETAILED DESCRIPTION

### Embodiments of the invention

In order to make the objectives, technical schemes and advantages of the present invention more apparent, the present invention is further described in detail. It should be understood that the specific embodiments described here are intended only to explain the present invention and are not intended to limit the present invention. It should be noted that the embodiments of the present invention and the features in the embodiments can be combined with each other without conflict.

The present invention provides an adjustable continuous-flow plasma disinfection and sterilization method and a corresponding disinfection and sterilization device.

According to the adjustable continuous-flow plasma disinfection and sterilization method of the present invention, a series of advanced oxidation media (such as ozone, singlet oxygen molecules, hydroxyl radicals, peroxide radicals and oxygen atoms) generated by high-energy plasmas produced in the working process of atmospheric-pressure plasmas, the heat of the plasmas and deep ultraviolet, extreme ultraviolet and even soft X-ray radiation generated in a de-excitation process, combined with collision between the plasmas and oxygen molecules in the air or the absorption of deep ultraviolet, extreme ultraviolet and even soft X-ray radiation by oxygen molecules in the air, serve as disinfection factors, to completely and thoroughly disinfect or sterilize a contacted medium to be treated. Combined with the new generation of atmospheric-pressure plasma technique, especially the surface coupled plasma technique, the method can realize efficient and large-scale continuous disinfection or sterilization.

Compared with the prior art, the inventor of the present application innovatively combines the atmospheric-pressure plasmas with an inlet-outlet pressure difference, which extends the working range of the atmospheric-pressure plasmas to a non-vacuum environment, realizes continuous plasma disinfection in a chamber with a certain specification and size, and allows sample feeding in an atmospheric or positive pressure environment, thus ensuring that continuous disinfection can be directly conducted in the atmospheric environment without a special vacuum chamber.

Based on this inventive concept, several different types of atmospheric-pressure plasma and pressure difference implementations are adopted in the present application to maintain the diffusion of plasmas. In addition, by controlling the movement form in the chamber, a microorganism carrier conveying unit can take different routes in the chamber, such that how long the microorganism carrier conveying unit stays in the disinfection chamber can be controlled, which further improves the disinfection efficiency, and allows microorganisms on a surface of the microorganism carrier conveying unit to be completely killed, so as to realize high-level disinfection or reach a sterilization level.

The atmospheric-pressure plasmas may be capacitive coupling plasmas, inductive coupling plasmas, high voltage DC arcs, high voltage AC arcs, microwave plasmas and surface coupling induced plasmas, preferably surface coupling induced plasmas, high voltage AC arcs and microwave plasmas. When ultraviolet and other radiation conditions are used as disinfection factors, the plasmas need to be excited to a high excited state to ensure that deep ultraviolet radiation and even soft X-ray radiation will be generated during radiative deexcitation, that is, high-efficiency plasma excitation can ensure high energy feeding efficiency and ideal plasma working parameters.

The plasma density of the atmospheric-pressure plasmas is 1.0×10³-1.0×10³⁰ plasmas per cubic centimeter, preferably 1.0×10⁵-1.0×10¹⁵ plasmas per cubic centimeter. The atom temperature of the atmospheric-pressure plasmas is 1.0×10⁰-1.0×10¹² K, preferably 3.5×10¹-1.0×10⁷ K. The electron temperature of the atmospheric-pressure plasmas is 1.0×10⁰-1.0×10¹² K, preferably 3.5×10¹-1.0×10⁷ K. In the process of plasma disinfection according to the present application, when a microorganism carrier is gas, liquid or multiphase flow during disinfection, the time the microorganism carrier stays in the disinfection and sterilization chamber is limited and disinfection needs to be conducted fast. Moreover, such carriers can withstand high-density plasma bombardment, so the excited state of the plasmas needs to be as high as possible, that is, the plasmas need to have a high degree of ionization, high density, and high electron temperature and atom temperature, so as to ensure a good disinfection effect. However, when the microorganism carrier is a solid carrier with a conveying movement mechanism during disinfection, the resistance to plasma bombardment is weak, but the solid carrier can stay in the chamber longer, that is, long-term action by low-density plasmas is required, so the plasmas need to have low density and low electron temperature and atom temperature. Therefore, for plasma disinfection, the applicable plasma density, atom temperature and electronic temperature vary with disinfection objects, so a wide adjustable range is required.

Microorganisms carried by the microorganism carrier include bacteria (and spores thereof), archaea, fungi, actinomycetes, protozoa, algae, viruses, mycoplasma, chlamydia, viroids, virusoids and prions. The microorganism killing rate ranges from 90% to 99.9999%. As for the disinfection method of the present application, instead of adopting chemical factors used in the traditional disinfection process as disinfection factors, disinfection is mainly realized through ultraviolet irradiation and ion irradiation, the disinfection intensity of which is high enough to efficiently decompose most gaseous organic substances into CHₓ fragments. Therefore, various common microorganisms can be effectively killed, and the method has a good disinfection and sterilization effect on typical indicator bacteria, such as Bacillus stearothermophilus spores, Bacillus subtilis spores, and Staphylococcus albus. Therefore, it is considered that the method is a broad-spectrum disinfection method, and has a microorganism killing rate high enough to realize high-level disinfection or reach a sterilization level.

The working medium gas in 1S1 includes one or a mixture of hydrogen, oxygen, nitrogen, artificial air, helium, neon, argon, krypton, xenon, chlorine, fluorine, bromine vapor, hydrogen fluoride, hydrogen chloride, hydrogen iodide, hydrogen bromide, nitrogen dioxide, nitrous oxide, nitrogen trifluoride, carbon monoxide, carbon dioxide, ammonia, sulfur hexafluoride, carbon tetrafluoride, silane gas, germane or organic gas, preferably hydrogen, oxygen, nitrogen, argon, helium, ammonia and carbon tetrafluoride. The working medium has a wide range of ionization energy, and various excimer excitation modes are allowed in a working medium mixture, so the plasma density required above can be realized.

The power of the plasma power source in 1S1 is 0.5 W-100 kW, preferably 1 w-5 kW. Selecting the said power range can avoid excessive energy consumption in the disinfection process.

The way of gradually adjusting the input power in 1S2 includes: first increasing the power and then decreasing the power to the target power, or gradually adding the power to the target power. The two adjustment modes have different effects for different power sources. When using the adjustment mode of first increasing the power and then decreasing the power to the target power, the energy consumption of the disinfection device will be increased and damage to parts of the disinfection device will be caused, but the formed plasmas have good stability and are easy to maintain. When using the adjustment mode of gradually adding the power to the target power, the energy consumption is relatively low, and the service life of the whole machine can be prolonged. However, the formed plasmas are poor in stability and tend to extinguish in work. Therefore, the adjustment mode should be selected according to specific working conditions.

The inlet-outlet pressure difference of the disinfection and sterilization chamber is 0 Pa-120 Mpa, preferably 0-200000 Pa. The inlet pressure of the disinfection and sterilization chamber is 0.0000001 Pa- 120 Mpa, preferably 100000-200000 Pa. The outlet pressure of the disinfection and sterilization chamber is 0.0000001 Pa-120 Mpa, preferably 0.01-100000 Pa. Generally, the atmospheric pressure is 100000 Pa, i.e. 0.1 Mpa. Under the said inlet pressure and outlet pressure, the pressure difference of the disinfection and sterilization chamber can be controlled within the atmospheric pressure range, so that the whole device can perform disinfection openly under atmospheric pressure, and the mean free path of the plasmas can be further extended, thus broadening the working range and shortening working hours.

The outlet pressure adjustment module in 2S1 includes one or more of a vortex fan, a centrifugal fan, an axial fan, a negative pressure Roots blower, a venturi tube, an air compressor, a gas decompression pump, a piston compressor, a jet vacuum pump, a screw vacuum pump, a liquid ring vacuum pump, a rotary vane vacuum pump, a claw vacuum pump, a Roots vacuum pump, a reciprocating vacuum pump, a molecular pump, a diffusion pump, an ion transport pump, an adsorption pump, a sublimation pump or a cryogenic pump, preferably one or more of a vortex fan, a negative pressure Roots blower, a venturi tube, a piston compressor, a jet vacuum pump or a liquid ring vacuum pump. The said outlet pressure adjustment module can be used to meet the aforementioned outlet pressure requirements, and has low requirements for working conditions and high tolerance, thus meeting the functional requirements of the present invention.

The inlet pressure adjustment module in 2S2 includes one or more of a vortex fan, a centrifugal fan, an axial fan, a Roots blower, a venturi tube, a gas booster pump, a piston compressor or a gas cylinder, preferably one or more of a vortex fan, a gas booster pump or a centrifugal fan. The said inlet pressure adjustment module can be used to meet the aforementioned inlet pressure requirements, and has low energy consumption and high efficiency, thus meeting the functional requirements of the present invention.

The microorganism carrier in 3S1 may be an airflow carrier, a liquid flow carrier, a multiphase flow carrier or a solid carrier with a conveying movement mechanism. When the microorganism carrier is an airflow carrier, the movement speed thereof in the disinfection and sterilization chamber is measured as a flow rate of 0.0001-100000 m³/min, preferably 0.5-20 m³/min. When the microorganism carrier is a liquid flow carrier, the movement speed thereof in the disinfection and sterilization chamber is measured as a flow rate of 0.0001-100000 m³/min, preferably 0.5-10 m³/min. When the microorganism carrier is a multiphase flow carrier, the movement speed thereof in the disinfection and sterilization chamber is measured as a flow rate of 0.0001-100000 m³/min, preferably 0.5-20 m³/min. When the microorganism carrier is a solid carrier with a conveying movement mechanism, the movement speed thereof in the disinfection and sterilization chamber is measured as a linear speed of 0.0001-60 m/min, preferably 0.01-1 m/min. The microorganism carrier determines the work object of the present application. When the work object is fluid, i.e. airflow, liquid flow and multiphase flow, the adaptive flow rate is often high, and the microorganism carrier can quickly pass through the disinfection and sterilization chamber. When the work object is non-fluid, that is, a solid carrier with a conveying movement mechanism, the said working speed is selected because a high mechanical movement speed will impose a heavy load on a moving mechanical part, which tends to cause wear.

The movement form of the microorganism carriers in the disinfection and sterilization chamber in 3S1 includes one or more of linear movement, spiral movement, vortex movement, rotary movement, reciprocating movement or random movement, preferably one or more of linear movement, vortex movement, rotary movement or random movement. The said movement form can prolong the travel distance of the microorganism carrier in the disinfection and sterilization chamber, and avoid excessive pipe loss and bending loss which may cause unexpected pressure drop in the chamber.

The duration of stay that meets disinfection or sterilization requirements in the disinfection and sterilization chamber in 3S1 is 0.5 s-24 h, within which the expected disinfection effect can be achieved.

In addition, the present invention also relates to a disinfection and sterilization device corresponding to the disinfection and sterilization method. In order to make those having ordinary skill in the art better understand the scheme of the present invention, the present invention will be explained in further detail below with reference to Fig. 1.

Please refer to Fig. 1, which is a schematic diagram of a specific implementation of a disinfection and sterilization device provided by the present invention.

In a specific implementation, the present invention provides a disinfection and sterilization device, which includes:
a housing: including a top cover 1, frame plates 4&10&11&16, a frame 19, an access opening 9, and a fixing component 13; a disinfection and sterilization chamber unit: including a disinfection and sterilization chamber module 21 (rectangular chamber structure, including a surface coupling induced plasma source ignition module, a working medium inlet module, an optical measurement interface and an electrical measurement interface), and a temperature measurement interface 20; a pressure adjustment unit: including a fan 14 (with a pressure measurement interface and a flow measurement interface) as an outlet pressure adjustment module, a gas cylinder 15 (with a pressure measurement interface) as an inlet pressure adjustment module, and a flow measurement interface 8; an atmospheric-pressure plasma unit: including a plasma source power module and power feedback measurement interface 17 and a working medium inlet module 6; a temperature control unit: including a temperature control unit 5; a microorganism carrier conveying unit: including a fluid conveying module 7; a state measurement and control unit: including control panels 2&3 and a master control module 18.

When the disinfection and sterilization device works, the microorganism carrier to be disinfected is delivered to the disinfection and sterilization chamber module 21 in the disinfection and sterilization chamber unit through the fluid conveying module 7 in the microorganism carrier delivery unit. Meanwhile, the working pressure of the disinfection and sterilization chamber module 21 is adjusted by the fan 14 (with the pressure measurement interface and the flow measurement interface) serving as the outlet pressure adjustment module and the gas cylinder 15 (with the pressure measurement interface) serving as the inlet pressure adjustment module in the pressure adjustment unit. The disinfection and sterilization chamber module 21 contains an ignition module (not shown) of the atmospheric-pressure plasma unit, air intake of the ignition module is realized through the working medium inlet module 6, and power input of the ignition module is realized through the plasma source power module and power feedback measurement interface 17. The working temperature of the whole machine is adjusted through the temperature control module 5. In the working process, the temperature measurement interface 20, the optical measurement interface and the electrical measurement interface on the disinfection and sterilization chamber module 21, the pressure measurement interface and the flow measurement interface of the outlet pressure adjustment module 14, the pressure measurement interface of the inlet pressure adjustment module 15, the flow measurement interface 8, and the power feedback measurement interface 17 of the plasma source power module provide various measurements to the master control module 18 of the state measurement and control unit at the same time, and the master control module 18 performs open-loop or closed-loop adjustment of the controlled variable of each unit according to control requirements input by the control panels 2&3.

Compared with the prior art, the disinfection and sterilization device can work under atmospheric pressure, and can realize sterilization and disinfection in open, semi-closed and closed areas by continuously treating fluids such as aerosol and multiphase flow in the environment, thus directly solving the space limitation problem of plasma disinfection. In addition, for continuous surface sterilization and disinfection, solid carrier modules under atmospheric pressure can be used to realize continuous feeding and discharging, so that surface disinfection of surgical instruments, implants and other materials can be carried out in large quantities. Other related devices satisfying the disinfection and sterilization methods involved in the present application also have corresponding beneficial effects. Please refer to the prior art for other related devices, which will not be described in detail here.

The scheme of the present invention will be further explained with specific embodiments below.

### Embodiment 1

The disinfection and sterilization device was turned on, argon was introduced into the ignition module of the atmospheric-pressure plasma unit in the disinfection and sterilization chamber module 21 through the working medium inlet module 6 until the atmosphere of the plasma unit was replaced with argon, and then the plasma power source 17 started to supply energy to the plasma unit. At the beginning, 1500 W was input through the control panels 2&3 as plasma ignition power, and the output power of the plasma source 17 was adjusted by the master control module 18. After the input power was completed and a plasma torch was formed, the plasmas were stabilized by adjusting the power to 1000 W through the control panels 2&3. Plasma parameters were measured through the optical measurement interface and the electrical measurement interface on the disinfection and sterilization chamber module 21, and it was determined that the plasma density was 5×10¹² plasmas per cubic centimeter, the atom temperature was 4.3×10⁵ K, and the electron temperature was 2.0×10⁴ K.

After the plasmas were stabilized, the outlet pressure of the disinfection and sterilization chamber module 21 was adjusted to 50000 Pa by the vortex fan 14 (with the pressure measurement interface and the flow measurement interface) serving as the outlet pressure adjustment module in the pressure adjustment unit, and the inlet pressure of the disinfection and sterilization chamber module 21 was adjusted to 100000 Pa by the gas cylinder 15 (with the pressure measurement interface) serving as the inlet pressure adjustment module. As pressure adjustment, the inlet-outlet pressure difference of the disinfection and sterilization chamber module 21 was 50000 Pa. Staphylococcus albus 8320 was used as the tested microorganism, and an air carrier was used as the microorganism carrier, which were sent into the disinfection and sterilization chamber module 21 through the fluid conveying module 7. The overall flow was measured through the flow measurement interface of the outlet pressure module 14, the flow rate of the air carrier in the disinfection and sterilization chamber 21 was controlled to be 1 m³/min, the air carrier was made to move linearly in the disinfection and sterilization chamber 21 and stay in the disinfection and sterilization chamber for 5 s, and finally the air carrier left the disinfection and sterilization chamber 21 to complete disinfection. The disinfected airflow was acquired for culture testing. Compared with a blank control group, the killing rate of staphylococcus albus 8320 in this experimental group reached 99.95%.

### Embodiment 2

Surface coupling induced plasmas were used as the atmospheric-pressure plasmas, the plasma density was 3.7×10¹¹ plasmas per cubic centimeter, the atom temperature was 4.3×10⁵ K, and the electron temperature was 2.0×10⁴ K. Argon was introduced into the working medium inlet module of the atmospheric-pressure plasma unit as the working medium gas until the atmosphere of the plasma unit was replaced with argon, and then the plasma power source started to supply energy to the plasma unit. At the beginning, the plasma ignition power was set to 800 W. After a plasma torch was formed, the power was gradually reduced to the target power 500 W to maintain the plasmas.

In the pressure adjustment unit connected to the disinfection and sterilization chamber, the outlet pressure adjustment unit used a liquid ring vacuum pump to adjust the outlet pressure, and the inlet pressure adjustment unit used a vortex fan to adjust the inlet pressure. After ignition, on the basis of ensuring that the plasmas were kept stable, the liquid ring vacuum pump was turned on to adjust the outlet pressure of the disinfection and sterilization chamber to 2000 Pa, and then the vortex fan was turned on to adjust the inlet pressure of the disinfection and sterilization chamber to 0.1 MPa. As pressure adjustment, the inlet-outlet pressure difference of the disinfection and sterilization chamber was 98000 Pa. Staphylococcus albus 8320 was used as the tested microorganism, and an air carrier was used as the microorganism carrier. The air carrier was sent into the disinfection and sterilization chamber for disinfection, and the flow rate of the air carrier passing through the disinfection and sterilization chamber was controlled to be 1 m³/min. The air carrier moved in a vortex motion in the disinfection and sterilization chamber, stayed in the disinfection and sterilization chamber for 20 s, and finally left the disinfection and sterilization chamber to complete disinfection. The disinfected airflow was acquired for culture testing. Compared with a blank control group, the killing rate of staphylococcus albus 8320 in this experimental group reached 99.9999%.

### Embodiment 3

Inductive coupling plasmas were used as the atmospheric-pressure plasmas, the plasma density was 3.5×10¹² plasmas per cubic centimeter, the atom temperature was 5×10⁵ K, and the electron temperature was 3.2×10⁴ K. Artificial air was introduced into the working medium inlet module of the atmospheric-pressure plasma unit as the working medium gas until the atmosphere of the plasma unit was replaced with artificial air, and then the plasma power source started to supply energy to the plasma unit. At the beginning, the plasma ignition power was set to 1000 W. After a plasma torch was formed, the power was gradually reduced to the target power 800 W to maintain the plasmas.

In the pressure adjustment unit connected to the disinfection and sterilization chamber, the outlet pressure adjustment unit used a liquid ring vacuum pump to adjust the outlet pressure, and the inlet pressure adjustment unit used a Roots blower to adjust the inlet pressure. After ignition, on the basis of ensuring that the plasmas were kept stable, the liquid ring vacuum pump was turned on to adjust the outlet pressure of the disinfection and sterilization chamber to 4000 Pa, and then the Roots blower was turned on to adjust the inlet pressure of the disinfection and sterilization chamber to 0.1 MPa. As pressure adjustment, the inlet-outlet pressure difference of the disinfection and sterilization chamber was 96000 Pa. A/California/07/2009 subtype cold-adapted attenuated vaccine strain of swine flu was used as the tested microorganism, and a solid carrier with a conveying movement mechanism was used as the microorganism carrier. The solid carrier was sent into the disinfection and sterilization chamber for disinfection, and the linear speed of the air carrier passing through the disinfection and sterilization chamber was controlled to be 1 m/min. The solid carrier moved linearly in the disinfection and sterilization chamber, stayed in the disinfection and sterilization chamber for 60 s, and finally left the disinfection and sterilization chamber to complete disinfection. After disinfection, culture testing was conducted on the surface of the solid carrier. Compared with a blank control group, the inactivation rate of the A/California/07/2009 subtype cold-adapted attenuated vaccine strain of swine flu in this experimental group reached 99.9991%.

### Embodiment 4

Microwave plasmas were used as the atmospheric-pressure plasmas, the plasma density was 4×10¹² plasmas per cubic centimeter, the atom temperature was 5×10⁵ K, and the electron temperature was 5×10⁴ K. Nitrogen was introduced into the working medium inlet module of the atmospheric-pressure plasma unit as the working medium gas until the atmosphere of the plasma unit was replaced with nitrogen, and then the plasma power source started to supply energy to the plasma unit. At the beginning, the plasma ignition power was set to 1000 W. After a plasma torch was formed, the power was gradually reduced to the target power 800 W to maintain the plasmas.

In the pressure adjustment unit connected to the disinfection and sterilization chamber, the outlet pressure adjustment unit used a liquid ring vacuum pump to adjust the outlet pressure, and the inlet pressure adjustment unit used a centrifugal fan to adjust the inlet pressure. After ignition, on the basis of ensuring that the plasmas were kept stable, the liquid ring vacuum pump was turned on to adjust the outlet pressure of the disinfection and sterilization chamber to 5000 Pa, and then the centrifugal fan was turned on to adjust the inlet pressure of the disinfection and sterilization chamber to 0.1 MPa. As pressure adjustment, the inlet-outlet pressure difference of the disinfection and sterilization chamber was 95000 Pa. Mycobacterium tuberculosis ATCC25177 was used as the tested microorganism, and a liquid carrier was used as the microorganism carrier. The liquid carrier was sent into the disinfection and sterilization chamber for disinfection, and the flow rate of the air carrier passing through the disinfection and sterilization chamber was controlled to be 1.5 m³/min. The liquid carrier moved in a vortex motion in the disinfection and sterilization chamber, stayed in the disinfection and sterilization chamber for 100 s, and finally left the disinfection and sterilization chamber to complete disinfection. The disinfected liquid flow was acquired for culture testing. Compared with a blank control group, the killing rate of mycobacterium tuberculosis ATCC25177 in this experimental group reached 99.999%.

### Embodiment 5

Surface coupling induced plasmas were used as the atmospheric-pressure plasmas, the plasma density was 3.2×10¹⁵ plasmas per cubic centimeter, the atom temperature was 3.4×10⁷ K, and the electron temperature was 3.5×10⁵ K. Helium was introduced into the working medium inlet module of the atmospheric-pressure plasma unit as the working medium gas until the atmosphere of the plasma unit was replaced with helium, and then the plasma power source started to supply energy to the plasma unit. At the beginning, the plasma ignition power was set to 5 kW. After a plasma torch was formed, the power was gradually reduced to the target power 3 kW to maintain the plasmas.

In the pressure adjustment unit connected to the disinfection and sterilization chamber, the outlet pressure adjustment unit used a screw vacuum pump to adjust the outlet pressure, and the inlet pressure adjustment unit used a gas booster pump to adjust the inlet pressure. After ignition, on the basis of ensuring that the plasmas were kept stable, the screw vacuum pump was turned on to adjust the outlet pressure of the disinfection and sterilization chamber to 5000 Pa, and then the gas booster pump was turned on to adjust the inlet pressure of the disinfection and sterilization chamber to 0.2 MPa. As pressure adjustment, the inlet-outlet pressure difference of the disinfection and sterilization chamber was 195000 Pa. Fusarium moniliforme BNCC186247 was used as the tested microorganism, and a multiphase flow carrier was used as the microorganism carrier. The multiphase flow carrier was sent into the disinfection and sterilization chamber for disinfection, and the flow rate of the air carrier passing through the disinfection and sterilization chamber was controlled to be 1.5 m³/min. The multiphase flow carrier moved linearly in the disinfection and sterilization chamber, stayed in the disinfection and sterilization chamber for 1 s, and finally left the disinfection and sterilization chamber to complete disinfection. The disinfected multiphase flow was acquired for culture testing. Compared with a blank control group, the killing rate of fusarium moniliforme BNCC 186247 in this experimental group reached 99.99%.

### Embodiment 6

Capacitive coupling plasmas were used as the atmospheric-pressure plasmas, the plasma density was 7.1×10¹² plasmas per cubic centimeter, the atom temperature was 5.6×10⁵ K, and the electron temperature was 5.5×10⁴ K. Carbon dioxide was introduced into the working medium inlet module of the atmospheric-pressure plasma unit as the working medium gas until the atmosphere of the plasma unit was replaced with carbon dioxide, and then the plasma power source started to supply energy to the plasma unit. At the beginning, the plasma ignition power was set to 1200 W. After a plasma torch was formed, the power was gradually reduced to the target power 800 W to maintain the plasmas.

In the pressure adjustment unit connected to the disinfection and sterilization chamber, the outlet pressure adjustment unit used a jet vacuum pump to adjust the outlet pressure, and the inlet pressure adjustment unit used an air compressor to adjust the inlet pressure. After ignition, on the basis of ensuring that the plasmas were kept stable, the jet vacuum pump was turned on to adjust the outlet pressure of the disinfection and sterilization chamber to 5000 Pa, and then the air compressor was turned on to adjust the inlet pressure of the disinfection and sterilization chamber to 0.3 MPa. As pressure adjustment, the inlet-outlet pressure difference of the disinfection and sterilization chamber was 295000 Pa. Porcine circovirus type 2 was used as the tested microorganism, and an air carrier was used as the microorganism carrier. The air carrier was sent into the disinfection and sterilization chamber for disinfection, and the flow rate of the air carrier passing through the disinfection and sterilization chamber was controlled to be 2 m³/min. The air carrier moved randomly in the disinfection and sterilization chamber, stayed in the disinfection and sterilization chamber for 10 h, and finally left the disinfection and sterilization chamber to complete disinfection. The disinfected airflow was acquired for culture testing. Compared with a blank control group, the inactivation rate of porcine circovirus type 2 in this experimental group reached 99.98%.

### Embodiment 7

Surface coupling induced plasmas were used as the atmospheric-pressure plasmas, the plasma density was 7.1×10¹² plasmas per cubic centimeter, the atom temperature was 5.6×10⁵ K, and the electron temperature was 5.5×10⁴ K. Carbon dioxide was introduced into the working medium inlet module of the atmospheric-pressure plasma unit as the working medium gas until the atmosphere of the plasma unit was replaced with carbon dioxide, and then the plasma power source started to supply energy to the plasma unit. At the beginning, the plasma ignition power was set to 1200 W. After a plasma torch was formed, the power was gradually reduced to the target power 800 W to maintain the plasmas.

In the pressure adjustment unit connected to the disinfection and sterilization chamber, the outlet pressure adjustment unit used a negative pressure Roots blower to adjust the outlet pressure, and the inlet pressure adjustment unit used an air compressor to adjust the inlet pressure. After ignition, on the basis of ensuring that the plasmas were kept stable, the negative pressure Roots blower was turned on to adjust the outlet pressure of the disinfection and sterilization chamber to 5000 Pa, and then the air compressor was turned on to adjust the inlet pressure of the disinfection and sterilization chamber to 0.2 MPa. As pressure adjustment, the inlet-outlet pressure difference of the disinfection and sterilization chamber was 195000 Pa. Bacillus subtilis var. niger ATCC9732 spore was used as the tested microorganism, and an air carrier was used as the microorganism carrier. The air carrier was sent into the disinfection and sterilization chamber for disinfection, and the flow rate of the air carrier passing through the disinfection and sterilization chamber was controlled to be 0.2 m³/min. The air carrier moved randomly in the disinfection and sterilization chamber, stayed in the disinfection and sterilization chamber for 1 h, and finally left the disinfection and sterilization chamber to complete disinfection. The disinfected airflow was acquired for culture testing. Compared with a blank control group, the killing rate of bacillus subtilis var. niger ATCC9732 in this experimental group reached 99.9999%.

### Embodiment 8

Capacitive coupling plasmas were used as the atmospheric-pressure plasmas, the plasma density was 7.2×10¹² plasmas per cubic centimeter, the atom temperature was 5.5×10⁵ K, and the electron temperature was 5.3×10⁴ K. Nitrogen was introduced into the working medium inlet module of the atmospheric-pressure plasma unit as the working medium gas until the atmosphere of the plasma unit was replaced with nitrogen, and then the plasma power source started to supply energy to the plasma unit. At the beginning, the plasma ignition power was set to 1200 W. After a plasma torch was formed, the power was gradually reduced to the target power 800 W to maintain the plasmas.

In the pressure adjustment unit connected to the disinfection and sterilization chamber, the outlet pressure adjustment unit used a liquid ring vacuum pump to adjust the outlet pressure, and the inlet pressure adjustment unit used a piston compressor to adjust the inlet pressure. After ignition, on the basis of ensuring that the plasmas were kept stable, the liquid ring vacuum pump was turned on to adjust the outlet pressure of the disinfection and sterilization chamber to 5000 Pa, and then the piston compressor was turned on to adjust the inlet pressure of the disinfection and sterilization chamber to 0.2 MPa. As pressure adjustment, the inlet-outlet pressure difference of the disinfection and sterilization chamber was 195000 Pa. The attenuated vaccine of H1N1 subtype influenza virus was used as the tested microorganism, and a liquid phase flow carrier was used as the microorganism carrier. The liquid phase flow carrier was sent into the disinfection and sterilization chamber for disinfection, and the flow rate of the air carrier passing through the disinfection and sterilization chamber was controlled to be 2 m³/min. The air carrier moved in a vortex motion in the disinfection and sterilization chamber, stayed in the disinfection and sterilization chamber for 100 s, and finally left the disinfection and sterilization chamber to complete disinfection. The disinfected airflow was acquired for culture testing. Compared with a blank control group, the inactivation rate of the attenuated vaccine of H1N1 subtype influenza virus in this experimental group reached 99.91%.

### Embodiment 9

Capacitive coupling plasmas were used as the atmospheric-pressure plasmas, the plasma density was 7.2×10¹² plasmas per cubic centimeter, the atom temperature was 5.5×10⁵ K, and the electron temperature was 5.3×10⁴ K. Nitrogen was introduced into the working medium inlet module of the atmospheric-pressure plasma unit as the working medium gas until the atmosphere of the plasma unit was replaced with nitrogen, and then the plasma power source started to supply energy to the plasma unit. At the beginning, the plasma ignition power was set to 1200 W. After a plasma torch was formed, the power was gradually reduced to the target power 800 W to maintain the plasmas.

In the pressure adjustment unit connected to the disinfection and sterilization chamber, the outlet pressure adjustment unit used a liquid ring vacuum pump, a Roots vacuum pump and a molecular pump to adjust the outlet pressure, and the inlet pressure adjustment unit used a vortex fan to adjust the inlet pressure. After ignition, on the basis of ensuring that the plasmas were kept stable, the liquid ring vacuum pump, the Roots vacuum pump and the molecular pump were turned on to adjust the outlet pressure of the disinfection and sterilization chamber to 0.00001 Pa, and then an axial flow fan was turned on to adjust the inlet pressure of the disinfection and sterilization chamber to 0.1 MPa. As pressure adjustment, the inlet-outlet pressure difference of the disinfection and sterilization chamber was 99999.99999 Pa. Bacillus stearothermophilus ATCC7953 spore was used as the tested microorganism, and a solid carrier with a conveying movement mechanism was used as the microorganism carrier. The solid carrier was sent into the disinfection and sterilization chamber for disinfection, and the linear speed of the air carrier passing through the disinfection and sterilization chamber was controlled to be 1 m/min. The solid carrier moved linearly in the disinfection and sterilization chamber, stayed in the disinfection and sterilization chamber for 40 min, and finally left the disinfection and sterilization chamber to complete disinfection. After disinfection, culture testing was conducted on the surface of the solid carrier. Compared with a blank control group, the killing rate of bacillus stearothermophilus ATCC7953 spore in this experimental group reached 99.9999%.

### Embodiment 10

Capacitive coupling plasmas were used as the atmospheric-pressure plasmas, the plasma density was 7.1×10¹² plasmas per cubic centimeter, the atom temperature was 5.6×10⁵ K, and the electron temperature was 5.5×10⁴ K. Nitrogen was introduced into the working medium inlet module of the atmospheric-pressure plasma unit as the working medium gas until the atmosphere of the plasma unit was replaced with nitrogen, and then the plasma power source started to supply energy to the plasma unit. At the beginning, the plasma ignition power was set to 1200 W. After a plasma torch was formed, the power was gradually reduced to the target power 800 W to maintain the plasmas.

In the pressure adjustment unit connected to the disinfection and sterilization chamber, the outlet pressure adjustment unit used an axial flow fan to adjust the outlet pressure, and the inlet pressure adjustment unit used an axial flow fan to adjust the inlet pressure. After ignition, on the basis of ensuring that the plasmas were kept stable, the axial flow fan was turned on to adjust the outlet pressure of the disinfection and sterilization chamber to 95000 Pa, and then the axial flow fan was turned on to adjust the inlet pressure of the disinfection and sterilization chamber to 0.1 MPa. As pressure adjustment, the inlet-outlet pressure difference of the disinfection and sterilization chamber was 5000 Pa. Aspergillus flavus BNCC185691 was used as the tested microorganism, and a liquid flow carrier was used as the microorganism carrier. The liquid flow carrier was sent into the disinfection and sterilization chamber for disinfection, and the flow rate of the air carrier passing through the disinfection and sterilization chamber was controlled to be 2 m³/min. The liquid flow carrier moved in a rotary motion in the disinfection and sterilization chamber, stayed in the disinfection and sterilization chamber for 20 min, and finally left the disinfection and sterilization chamber to complete disinfection. The disinfected airflow was acquired for culture testing. Compared with a blank control group, the killing rate of aspergillus flavus BNCC185691 in this experimental group reached 99.6%.

### Embodiment 11

High voltage AC arcs were used as the atmospheric-pressure plasmas, the plasma density was 5.5×10¹² plasmas per cubic centimeter, the atom temperature was 4.4×10⁵ K, and the electron temperature was 3.5×10⁴ K. Carbon dioxide was introduced into the working medium inlet module of the atmospheric-pressure plasma unit as the working medium gas until the atmosphere of the plasma unit was replaced with carbon dioxide, and then the plasma power source started to supply energy to the plasma unit. At the beginning, the plasma ignition power was set to 1000 W. After a plasma torch was formed, the power was gradually reduced to the target power 650 W to maintain the plasmas.

In the pressure adjustment unit connected to the disinfection and sterilization chamber, the outlet pressure adjustment unit used a venturi tube to adjust the outlet pressure, and the inlet pressure adjustment unit used a centrifugal fan to adjust the inlet pressure. After ignition, on the basis of ensuring that the plasmas were kept stable, the venturi tube was turned on to adjust the outlet pressure of the disinfection and sterilization chamber to 50000 Pa, and then the centrifugal fan was turned on to adjust the inlet pressure of the disinfection and sterilization chamber to 0.2 MPa. As pressure adjustment, the inlet-outlet pressure difference of the disinfection and sterilization chamber was 150000 Pa. The vaccine of poliovirus type I was used as the tested microorganism, and a multiphase flow carrier was used as the microorganism carrier. The multiphase flow carrier was sent into the disinfection and sterilization chamber for disinfection, and the flow rate of the air carrier passing through the disinfection and sterilization chamber was controlled to be 20 m³/min. The airflow carrier moved in a vortex motion in the disinfection and sterilization chamber, stayed in the disinfection and sterilization chamber for 0.5 s, and finally left the disinfection and sterilization chamber to complete disinfection. The disinfected airflow was acquired for culture testing. Compared with a blank control group, the inactivation rate of the vaccine of poliovirus type I in this experimental group reached 91%.

### Embodiment 12

High voltage AC arcs were used as the atmospheric-pressure plasmas, the plasma density was 5.5×10¹² plasmas per cubic centimeter, the atom temperature was 4.4×10⁵ K, and the electron temperature was 3.5×10⁴ K. Carbon dioxide was introduced into the working medium inlet module of the atmospheric-pressure plasma unit as the working medium gas until the atmosphere of the plasma unit was replaced with carbon dioxide, and then the plasma power source started to supply energy to the plasma unit. At the beginning, the plasma ignition power was set to 1000 W. After a plasma torch was formed, the power was gradually reduced to the target power 650 W to maintain the plasmas.

In the pressure adjustment unit connected to the disinfection and sterilization chamber, the outlet pressure adjustment unit used a liquid ring vacuum pump to adjust the outlet pressure, and the inlet pressure adjustment unit used a centrifugal fan to adjust the inlet pressure. After ignition, on the basis of ensuring that the plasmas were kept stable, the liquid ring vacuum pump was turned on to adjust the outlet pressure of the disinfection and sterilization chamber to 5000 Pa, and then the vortex fan was turned on to adjust the inlet pressure of the disinfection and sterilization chamber to 0.2 MPa. As pressure adjustment, the inlet-outlet pressure difference of the disinfection and sterilization chamber was 195000 Pa. Staphylococcus aureus ATCC6538 was used as the tested microorganism, and a multiphase flow carrier was used as the microorganism carrier. The multiphase flow carrier was sent into the disinfection and sterilization chamber for disinfection, and the flow rate of the air carrier passing through the disinfection and sterilization chamber was controlled to be 1.5 m³/min. The airflow carrier moved in a vortex motion in the disinfection and sterilization chamber, stayed in the disinfection and sterilization chamber for 400 s, and finally left the disinfection and sterilization chamber to complete disinfection. The disinfected airflow was acquired for culture testing. Compared with a blank control group, the killing rate of staphylococcus aureus ATCC6538 in this experimental group reached 99.99%.

### Embodiment 13

High voltage AC arcs were used as the atmospheric-pressure plasmas, the plasma density was 5.5×10¹² plasmas per cubic centimeter, the atom temperature was 4.4×10⁵ K, and the electron temperature was 3.5×10⁴ K. Carbon dioxide was introduced into the working medium inlet module of the atmospheric-pressure plasma unit as the working medium gas until the atmosphere of the plasma unit was replaced with carbon dioxide, and then the plasma power source started to supply energy to the plasma unit. At the beginning, the plasma ignition power was set to 1000 W. After a plasma torch was formed, the power was gradually reduced to the target power 650 W to maintain the plasmas.

In the pressure adjustment unit connected to the disinfection and sterilization chamber, the outlet pressure adjustment unit used a rotary vane vacuum pump to adjust the outlet pressure, and the inlet pressure adjustment unit used a piston compressor to adjust the inlet pressure. After ignition, on the basis of ensuring that the plasmas were kept stable, the rotary vane vacuum pump was turned on to adjust the outlet pressure of the disinfection and sterilization chamber to 5000 Pa, and then the piston compressor was turned on to adjust the inlet pressure of the disinfection and sterilization chamber to 0.2 MPa. As pressure adjustment, the inlet-outlet pressure difference of the disinfection and sterilization chamber was 195000 Pa. Escherichia coli ATCC8739 was used as the tested microorganism, and a solid carrier with a conveying movement mechanism was used as the microorganism carrier. The solid carrier was sent into the disinfection and sterilization chamber for disinfection, and the linear speed of the air carrier passing through the disinfection and sterilization chamber was controlled to be 0.5 m/min. The solid carrier moved in a vortex motion in the disinfection and sterilization chamber, stayed in the disinfection and sterilization chamber for 1 h, and finally left the disinfection and sterilization chamber to complete disinfection. After disinfection, culture testing was conducted on the surface of the solid carrier. Compared with a blank control group, the killing rate of Escherichia coli ATCC8739 in this experimental group reached 99.99%.

The above are only preferred embodiments of the present invention, and are not intended to limit the present invention. Any modification, equivalent replacement, improvement, etc. made within the spirit and principle of the present invention shall fall within the scope of protection of the present invention.

## Claims

1. An adjustable continuous-flow plasma disinfection and sterilization method, comprising:
(1) exciting atmospheric-pressure plasmas in a disinfection and sterilization chamber featuring an adjustable inlet-outlet pressure difference;
(2) adjusting the inlet-outlet pressure difference of the disinfection and sterilization chamber after the atmospheric-pressure plasmas become stable, so as to adjust the plasma density in the chamber;
(3) continuously feeding microorganism carriers to be disinfected and sterilized into the disinfection and sterilization chamber for disinfection and sterilization; and
(4) discharging the carriers out of the disinfection and sterilization chamber to complete disinfection or sterilization.

2. The adjustable continuous-flow plasma disinfection and sterilization method of claim 1, wherein the atmospheric-pressure plasmas comprise one or more of capacitive coupling plasmas, inductive coupling plasmas, high voltage DC arcs, high voltage AC arcs, microwave plasmas or surface coupling induced plasmas.

3. The adjustable continuous-flow plasma disinfection and sterilization method of claim 1, wherein the plasma density of the atmospheric-pressure plasmas is 1.0×10³-1.0×10³⁰ plasmas per cubic centimeter.

4. The adjustable continuous-flow plasma disinfection and sterilization method of claim 1, wherein the atom temperature of the atmospheric-pressure plasmas is 1.0×10⁰-1.0×10¹² K.

5. The adjustable continuous-flow plasma disinfection and sterilization method of claim 1, wherein the electron temperature of the atmospheric-pressure plasmas is 1.0×10⁰-1.0×10¹² K.

6. The adjustable continuous-flow plasma disinfection and sterilization method of claim 1, wherein microorganisms carried by the microorganism carrier comprise bacteria (and spores thereof), archaea, fungi, actinomycetes, protozoa, algae, viruses, mycoplasma, chlamydia, viroids, virusoids and prions.

7. The adjustable continuous-flow plasma disinfection and sterilization method of claim 1, wherein the microorganism killing rate ranges from 90% to 99.9999%.

8. The adjustable continuous-flow plasma disinfection and sterilization method of claim 1, wherein exciting atmospheric-pressure plasmas in a disinfection and sterilization chamber featuring an adjustable inlet-outlet pressure difference specifically comprises the following steps:
1S1. introducing a working medium gas into an atmospheric-pressure plasma unit, and after the atmospheric-pressure plasmas are saturated with the working medium gas, supplying energy to a plasma power source until the plasmas are successfully ignited to form a plasma torch; and
1S2. on the basis of ensuring that the plasmas are kept stable, gradually adjusting the input power of the plasma source until the input power of the plasmas reaches target power.

9. The adjustable continuous-flow plasma disinfection and sterilization method of claim 8, wherein the working medium gas in 1S1 comprises one or a mixture of hydrogen, oxygen, nitrogen, artificial air, helium, neon, argon, krypton, xenon, chlorine, fluorine, bromine vapor, hydrogen fluoride, hydrogen chloride, hydrogen iodide, hydrogen bromide, nitrogen dioxide, nitrous oxide, nitrogen trifluoride, carbon monoxide, carbon dioxide, ammonia, sulfur hexafluoride, carbon tetrafluoride, silane gas, germane or organic gas.

10. The adjustable continuous-flow plasma disinfection and sterilization method of claim 8, wherein the power of the plasma power source in 1S1 is 0.5 W-100 kW.

11. The adjustable continuous-flow plasma disinfection and sterilization method of claim 8, wherein the way of gradually adjusting the input power in 1S2 comprises: first increasing the power and then decreasing the power to the target power, or gradually adding the power to the target power.

12. The adjustable continuous-flow plasma disinfection and sterilization method of claim 8, wherein adjusting the inlet-outlet pressure difference of the disinfection and sterilization chamber after the atmospheric-pressure plasmas become stable, so as to adjust the plasma density in the chamber specifically comprises the following steps: 2S1: on the basis of ensuring that the plasmas are kept stable, adjusting an outlet pressure of the disinfection and sterilization chamber by means of an outlet pressure adjustment module in a pressure adjustment unit connected to the disinfection and sterilization chamber; and 2S2: on the basis of ensuring that the plasmas are kept stable, adjusting an inlet pressure of the disinfection and sterilization chamber by means of an inlet pressure adjustment module in the pressure adjustment unit of the disinfection and sterilization chamber.

13. The adjustable continuous-flow plasma disinfection and sterilization method of claim 12, wherein the inlet-outlet pressure difference of the disinfection and sterilization chamber is 0 Pa-120 MPa.

14. The adjustable continuous-flow plasma disinfection and sterilization method of claim 12, wherein the inlet pressure of the disinfection and sterilization chamber is 0. 0000001 Pa-120 Mpa.

15. The adjustable continuous-flow plasma disinfection and sterilization method of claim 12, wherein the outlet pressure of the disinfection and sterilization chamber is 0.0000001 Pa-120 Mpa.

16. The adjustable continuous-flow plasma disinfection and sterilization method of claim 12, wherein the outlet pressure adjustment module in 2S1 comprises one or more of a vortex fan, a centrifugal fan, an axial fan, a negative pressure Roots blower, a venturi tube, an air compressor, a gas decompression pump, a piston compressor, a jet vacuum pump, a screw vacuum pump, a liquid ring vacuum pump, a rotary vane vacuum pump, a claw vacuum pump, a Roots vacuum pump, a reciprocating vacuum pump, a molecular pump, a diffusion pump, an ion transport pump, an adsorption pump, a sublimation pump or a cryogenic pump.

17. The adjustable continuous-flow plasma disinfection and sterilization method of claim 12, wherein the inlet pressure adjustment module of the pressure adjustment unit in 2S2 comprises one or more of a vortex fan, a centrifugal fan, an axial fan, a Roots blower, a venturi tube, a gas booster pump, a piston compressor or a gas cylinder.

18. The adjustable continuous-flow plasma disinfection and sterilization method of claim 1, wherein continuously feeding microorganism carriers to be disinfected and sterilized into the disinfection and sterilization chamber for disinfection or sterilization specifically comprises the following steps: 3S1: feeding the microorganism carriers into the disinfection and sterilization chamber at a certain speed, and adjusting the movement speed and form of the microorganism carriers in the disinfection and sterilization chamber according to a size of the disinfection and sterilization chamber and the power of a plasma source, so as to ensure that the microorganism carriers stay in the disinfection and sterilization chamber for an amount of time that meets disinfection or sterilization requirements.

19. The adjustable continuous-flow plasma disinfection and sterilization method of claim 18, wherein the microorganism carrier in 3S1 comprises one or more of an airflow carrier, a liquid flow carrier, a multiphase flow carrier or a solid carrier with a conveying movement mechanism.

20. The adjustable continuous-flow plasma disinfection and sterilization method of claim 18, wherein when the microorganism carrier is an airflow carrier, the movement speed thereof in the disinfection and sterilization chamber is measured as a flow rate of 0.0001-100000 m³/min.

21. The adjustable continuous-flow plasma disinfection and sterilization method of claim 18, wherein when the microorganism carrier is a liquid flow carrier, the movement speed thereof in the disinfection and sterilization chamber is measured as a flow rate of 0.0001-100000 m³/min.

22. The adjustable continuous-flow plasma disinfection and sterilization method of claim 18, wherein when the microorganism carrier is a multiphase flow carrier, the movement speed thereof in the disinfection and sterilization chamber is measured as a flow rate of 0.0001-100000 m³/min.

23. The adjustable continuous-flow plasma disinfection and sterilization method of claim 18, wherein when the microorganism carrier is a solid carrier with a conveying movement mechanism, the movement speed thereof in the disinfection and sterilization chamber is measured as a linear speed of 0.0001-60 m/min.

24. The adjustable continuous-flow plasma disinfection and sterilization method of claim 18, wherein the movement form of the microorganism carriers in the disinfection and sterilization chamber in 3S1 comprises one or more of linear movement, spiral movement, vortex movement, rotary movement, reciprocating movement or random movement.

25. The adjustable continuous-flow plasma disinfection and sterilization method of claim 18, wherein the duration of stay that meets disinfection or sterilization requirements in the disinfection and sterilization chamber in 3S1 is 0.5 s - 24 h.

26. The adjustable continuous-flow plasma disinfection and sterilization method of claim 1, wherein discharging the carriers out of the disinfection and sterilization chamber after treatment, which marks the completion of disinfection or sterilization of the carriers according to different microorganism killing rate requirements, specifically comprises the following steps: 4S1: after the microorganism carriers have stayed in the disinfection and sterilization chamber for an amount of time that meets disinfection or sterilization requirements, discharging the microorganism carriers out of the disinfection and sterilization chamber to complete the disinfection process.

27. A disinfection and sterilization device capable of implementing the adjustable continuous-flow plasma disinfection and sterilization method of any one of claims 1-26.

28. The disinfection and sterilization device of claim 27, wherein the disinfection and sterilization device comprises a disinfection and sterilization chamber unit, a pressure adjustment unit, an atmospheric-pressure plasma unit, a microorganism carrier conveying unit, a state measurement and control unit and a temperature control unit.

29. The disinfection and sterilization device of claim 27, wherein the disinfection and sterilization chamber unit contains an ignition module of the atmospheric-pressure plasma unit, and an inlet and outlet of the disinfection and sterilization chamber unit communicate with the pressure adjustment unit, thereby realizing pressure adjustment of the disinfection and sterilization chamber.

30. The disinfection and sterilization device of claim 27, wherein the microorganism carrier conveying unit communicates with the pressure adjustment unit, such that microorganism carriers are fed into the disinfection and sterilization chamber under adjustable pressure environments and discharged after disinfection.

31. The disinfection and sterilization device of claim 27, wherein the temperature control unit is connected to the disinfection and sterilization chamber unit, the atmospheric-pressure plasma unit and the microorganism carrier conveying unit, so as to adjust the working temperature of the above units and realize constant temperature.

32. The disinfection and sterilization device of claim 27, wherein the state measurement and control unit controls the disinfection and sterilization chamber unit and the atmospheric-pressure plasma torch unit.

33. The disinfection and sterilization device of claim 27, wherein the state measurement and control unit controls the states of the microorganism carrier conveying unit and the pressure adjustment unit, so as to continuously measure, control and adjust the disinfection process.

34. The disinfection and sterilization device of claim 27, wherein the disinfection and sterilization chamber unit comprises a disinfection and sterilization chamber module, an optical measurement interface, an electrical measurement interface and a temperature measurement interface.

35. The disinfection and sterilization device of claim 27, wherein the structure of the disinfection and sterilization chamber module comprises one or more of a rectangular chamber structure, a cylindrical chamber structure, a spherical chamber structure, a conical chamber structure, an ellipsoidal chamber structure, a parabolic chamber structure, a hyperboloid chamber structure, a spiral chamber structure, a volute chamber structure or a special-shaped chamber structure.

36. The disinfection and sterilization device of claim 27, wherein the pressure adjustment unit comprises an outlet pressure adjustment module, an inlet pressure adjustment module, pressure measurement interfaces and flow measurement interfaces, wherein the outlet pressure adjustment module and the inlet pressure adjustment module are each provided with a pressure measurement interface and a flow measurement interface.

37. The disinfection and sterilization device of claim 27, wherein the atmospheric-pressure plasma unit comprises a plasma source power module, a working medium inlet module, an ignition module and a power feedback measurement interface, wherein the plasma source ignition module is arranged in the disinfection and sterilization chamber module, the working medium inlet module is installed at a rear end of the ignition module, and the plasma source power module is provided with the power feedback measurement interface and monitored by the state measurement and control unit.

38. The disinfection and sterilization device of claim 27, wherein the temperature control unit comprises a heat dissipation module and a heat exchange module, wherein the heat exchange module is connected to the disinfection and sterilization chamber unit, the atmospheric-pressure plasma unit and the microorganism carrier conveying unit to ensure that the heat generated during the operation of the above units is quickly transferred out, and meanwhile, the heat exchange module is connected to the heat dissipation module to ensure that the heat quickly contacts with the environment or a refrigerant/heat medium, thus controlling the temperature.

39. The disinfection and sterilization device of claim 27, wherein the microorganism carrier conveying unit comprises one or more of an airflow conveying module, or a liquid flow conveying module, or a multiphase flow conveying module or a solid carrier module with a conveying movement mechanism.

40. The disinfection and sterilization device of claim 27, wherein the state measurement and control unit comprises a control panel, a master control module, an optical measurement module, an electrical measurement module, a temperature measurement module, a pressure measurement module, a flow measurement module and a plasma source power feedback measurement module.
